# EUROPEAN PATENT APPLICATION

(11) **EP 4 644 407 A1**
(43) Date of publication of application: **05.11.2025**
(21) Application number: 22969661.2
(22) Date of filing: 29.12.2022
(51) Int. Cl.: C07K 14/00, C12N 15/00, C12N 5/10, C12Q 1/6869, G01N 27/447, G01N 33/68, A61K 47/42

(54) **NOVEL PORIN BCP34, AND MUTANT THEREOF AND USE THEREOF**

(71) Applicant: BGI Shenzhen, Shenzhen, Guangdong 518083 (CN); BGI Shenzhen Co., Ltd, Shenzhen, Guangdong 518083 (CN)
(72) Inventor: XU, Xun, Shenzhen, Guangdong 518083 (CN); JIANG, Nan, Shenzhen, Guangdong 518083 (CN); LIU, Huanhuan, Shenzhen, Guangdong 518083 (CN); WANG, Lele, Shenzhen, Guangdong 518083 (CN); GUO, Fei, Shenzhen, Guangdong 518083 (CN); ZENG, Tao, Shenzhen, Guangdong 518083 (CN); JI, Zhouxiang, Shenzhen, Guangdong 518083 (CN); LI, Yuxiang, Shenzhen, Guangdong 518083 (CN); DONG, Yuliang, Shenzhen, Guangdong 518083 (CN); ZHANG, Wenwei, Shenzhen, Guangdong 518083 (CN)
(74) Representative: Weickmann & Weickmann PartmbB
(86) International application number: PCT/CN2022/143298
(87) International publication number: WO 2024/138512

(57) **Abstract**

The present invention provides a novel porin BCP34, and a mutant thereof and a use thereof. Specifically, the wild-type porin BCP34 is a nonamer protein, and has a monomer of which the amino acid sequence is SEQ ID NO: 1; and the mutant of the monomer of the porin BCP34 comprises an amino acid sequence having at least 70%, 75%, 80%, 85%, 90%, 95%, or at least 99% identity to SEQ ID NO:1, and an amino acid sequence having mutations at one or more positions in the sensor region, a transmembrane region, the entrance, barrel inner wall, and the exit of the monomer of the wild-type porin BCP34. The wild-type porin BCP34 of the present invention has the capability of being applied to nanopore sequencing, and the mutant of the porin BCP34 obtained by optimization has higher library capture capability and nanopore sequencing accuracy.

## Description

### Technical Field

The present invention relates to the field of nanopore sequencing technology, and specifically to a novel porin BCP34, mutant thereof and use thereof.

### Background

Nanopore sequencing is a third-generation sequencing technology that has emerged in recent years. Its advantages such as long read length, high throughput, low cost and portability have brought disruptive changes to the gene sequencing industry. This technology is widely applied in fundamental theoretical research of life sciences and biomedical clinical practice. Nanopore sequencing technology is a sequencing technology based on electrical signals. This technology can be applied to DNA, RNA and protein sequencing at the same time. It records the continuous blocked electrical signals generated when the analytes pass through the nanopore proteins one by one in real time, and converts them into sequence information through analysis to achieve sequencing.

At present, Oxford Nanopore Technologies (UK) has developed a series of nanopore sequencers such as MinION, GridION and PromethION, and Qi Carbon Technology has also developed QNome-3841 nanopore sequencer. However, they still have major deficiencies in sequencing accuracy, throughput and chip stability, and cannot meet the ultimate needs of molecular biology research. Therefore, a single-molecule sequencer with high accuracy, high integration and high stability is needed in this field.

Nanopore sequencing requires that the sensor region inside the pore protein is sharp enough to have high spatial resolution in both the horizontal and vertical directions. Among the pore proteins studied, there are three main types of proteins that can be potentially used for sequencing: pore-forming toxin proteins produced by bacteria or other organisms that destroy the permeability of cell membranes; transporter proteins that serve as transport channels for various biological macromolecules and small molecules inside and outside cells; and viral connectors that provide genome transport channels when viruses infect hosts. At present, only a few natural proteins such *as Mycobacterium smegmatis* pore protein A (MspA) and curli-specific transport channel (CsgG) meet the sequencing requirements. However, in the in vitro expression and purification system of recombinant proteins, the pore stability of recombinant nanopore proteins may not meet the needs of single-molecule detector-related instrument products. Finding more nanopore proteins that meet the sequencing requirements is still an unresolved problem.

### Contents of the present invention

The purpose of the present invention is to provide a novel porin BCP34 and mutant thereof, and the use of the porin BCP34 and mutant thereof for nanopore sequencing and other related applications.

In the first aspect, the present invention provides a porin monomer, wherein the porin monomer is a protein having an amino acid sequence as set forth in SEQ ID NO:1 or a mutant thereof having the function of a porin monomer.

In the second aspect, the present invention provides a construct, wherein the construct comprises 7 to 11 porin monomers as described in the first aspect; preferably, the construct comprises 9 porin monomers as described in the first aspect.

In the third aspect, the present invention provides a protein pore, wherein the protein pore comprises 7 to 11 porin monomers as described in the first aspect; preferably, the protein pore comprises 9 porin monomers as described in the first aspect.

In the fourth aspect, the present invention provides a membrane, wherein the membrane has the protein pore as described in the third aspect.

In the fifth aspect, the present invention provides a complex for characterizing a target analyte, wherein the complex comprises the protein pore as described in the third aspect and a rate-controlling protein used in conjunction with the protein pore.

In the sixth aspect, the present invention provides a nucleic acid, wherein the nucleic acid encodes the porin monomer as described in the first aspect, the construct as described in the second aspect, the protein pore as described in the third aspect, or the complex as described in the fifth aspect.

In the seventh aspect, the present invention provides a vector or a host cell, wherein the vector or host cell comprises the nucleic acid as described in the sixth aspect.

In the eighth aspect, the present invention provides a method for producing a protein pore or polypeptide thereof, comprising transforming a host cell with the vector as described in the seventh aspect, and inducing the host cell to express the protein pore or polypeptide thereof.

In the ninth aspect, the present invention provides a method for determining the presence, absence, or one or more characteristics of a target analyte, the method comprising:
a. contacting the target analyte with a protein pore on the membrane as described in the fourth aspect, or the complex as described in the fifth aspect or a protein pore in the complex, so that the target analyte moves relative to the protein pore;
b. obtaining one or more measurement values when the target analyte moves relative to the protein pore, thereby determining the presence, absence, or one or more characteristics of the target analyte.

In the tenth aspect, the present invention provides a kit for determining the presence, absence, or one or more characteristics of a target analyte, the kit comprising the construct as described in the second aspect, the protein pore as described in the third aspect, the membrane as described in the fourth aspect, the complex as described in the fifth aspect, the nucleic acid as described in the sixth aspect, or the vector or host cell as described in the seventh aspect.

In the eleventh aspect, the present invention provides a device for determining the presence, absence or one or more characteristics of a target analyte, the device comprising the protein pore as described in the third aspect, the membrane as described in the fourth aspect, or the complex as described in the fifth aspect.

Using the above technical solution, the beneficial effects of the present invention are: the wild-type porin BCP34 has very low homology with the protein sequence of the prior art and has the ability to be applied to nanopore sequencing; the porin BCP34 mutant obtained by optimization has higher library capture ability and nanopore sequencing accuracy.

### Brief Description of the Drawings

Fig. 1 shows a side view of the predicted structure of BCP34.
Fig. 2 shows a top view of the predicted structure of BCP34.
Fig. 3 shows a schematic diagram of the sensor region of the predicted structure of BCP34.
Fig. 4 shows a schematic diagram of important amino acid residues in the sensor region of the predicted structure of BCP34.
Fig. 5 shows a schematic diagram of four charged and polar amino acid residues facing the membrane direction in the transmembrane region of the predicted structure of BCP34.
Fig. 6 shows a schematic diagram of key amino acid residues in the inlet region of the predicted structure of BCP34 monomer.
Fig. 7 shows a schematic diagram of charge distribution in the inlet region of the predicted structure of BCP34.
Fig. 8 shows a schematic diagram of the key amino acid residues in the barrel inner wall and the outlet region in the predicted structure of the BCP34 monomer.
Fig. 9 shows an SDS-PAGE result of BCP34 after purification in an example of the present invention.
Fig. 10 shows a schematic diagram of the sequencing library structure in an example of the present invention, wherein a: positive strand; b: antisense strand; c: double-stranded target fragment to be tested; d: helicase BCH105; e: single-stranded DNA with cholesterol.
Fig. 11 shows the nanopore biosensor current of wild-type BCP34 when voltages of 0.02V, 0.04V, 0.10V, 0.14V and 0.18V are applied in an example of the present invention.
Fig. 12 shows the nanopore biosensor current of BCP34 mutant 1 when voltages of 0.02V, 0.04V, 0.10V, 0.14V and 0.18V are applied in an example of the present invention.
Fig. 13 shows the current trace changes of library DNA passing through BCP34 mutant 1 under the action of an applied voltage of 0.14V in an example of the present invention.
Fig. 14 shows the local details of the current trace when BCP34 mutant 1 performs DNA sequencing under the action of an applied voltage of 0.14V in an example of the present invention.
Fig. 15 shows the capture results of QCDNA by two pore proteins, wherein Fig. 15A shows the capture results of QCDNA by BCP34 mutant 1 pore protein, and Fig. 15B shows the capture results of QCDNA by the prior art nanopore protein CSGG-Y51A/F56Q.

### Specific Models for Carrying Out the present invention

In the following, the present invention will be described in detail in conjunction with the accompanying drawings. It should be understood that the following description is only used to illustrate the present invention by way of example, and is not intended to limit the scope of the present invention. The scope of protection of the present invention shall be subject to the appended claims. In addition, those skilled in the art understand that the technical solution of the present invention can be modified without departing from the spirit and purpose of the present invention. If not specifically stated, the technical means used in the examples are conventional means known to those skilled in the art.

Unless otherwise specified, the scientific and technical terms used in the present application have the meanings commonly understood by those skilled in the art. In order to better understand the present invention, the definitions and explanations of the relevant terms are provided below.

The term "comprising" means that any listed elements must be included, and other elements may also be optionally included. "Consisting of..." means that all unlisted elements are not included. Examples defined by each of these terms are within the scope of the present invention.

"One or more positions" refers to 1, 2, 3, 4, 5, 6, 7, 8, 9, 10... or up to all positions. For example, one or more positions of 5 amino acids refer to 1, 2, 3, 4 or 5 positions.

The term "wild type" refers to a gene or gene product isolated from a naturally occurring source.

The term "amino acid" is used in its broadest sense and is meant to include organic compounds containing amine (NH₂) and carboxyl (COOH) functional groups and side chains (e.g., R groups) unique to each amino acid.

The terms "protein", "polypeptide" and "peptide" are used interchangeably herein to refer to polymers of amino acid residues as well as variants and synthetic analogs of amino acid residues. Thus, these terms apply to amino acid polymers in which one or more amino acid residues are synthetic non-naturally occurring amino acids, such as chemical analogs of corresponding naturally occurring amino acids, as well as to naturally occurring amino acid polymers. Polypeptides may also undergo maturation or post-translational modification processes, which may include, but are not limited to, glycosylation, proteolytic cleavage, lipidation, signal peptide cleavage, propeptide cleavage, phosphorylation, and the like.

The term "nucleic acid" refers to a single-stranded or double-stranded covalently linked sequence of nucleotides in which the 3' and 5' ends of each nucleotide are linked by a phosphodiester bond. Nucleic acids may include DNA and RNA, and may be prepared synthetically in vitro or isolated from natural sources. The length of a nucleic acid is generally expressed in terms of the number of base pairs for double-stranded polynucleotides, or in terms of the number of nucleotides in the case of single-stranded polynucleotides. Polynucleotides less than about 40 nucleotides in length are generally referred to as "oligonucleotides". The term "polynucleotide" refers to a macromolecule comprising two or more nucleotides, which may be single-stranded or double-stranded. The polynucleotide or nucleic acid may comprise any combination of any nucleotides. The nucleotides may be naturally occurring or artificially synthesized.

The term "sequence identity" refers to the degree of sequence identity on a nucleotide-by-nucleotide or amino acid-by-amino acid basis over a comparison window. The "percentage of sequence identity" can be calculated by comparing two sequences in a comparison window, determining the number of positions at which the same nucleic acid base (e.g., A, T, C, G, U) or the same amino acid residue (e.g., Ala, Pro, Ser, Thr, Gly, Val, Leu, Ile, Phe, Tyr, Trp, Lys, Arg, His, Asp, Glu, Asn, Gln, Cys, and Met) is present in the two sequences to obtain the number of matching positions, dividing the number of matching positions by the total number of positions in the comparison window (i.e., the window size), and multiplying the result by 100% to obtain the "percentage of sequence identity". To calculate the "percentage of sequence identity", it can be achieved in a variety of ways known in the art, for example, using publicly available computer software such as BLAST, BLAST-2, ALIGN or Megalign (DNASTAR) software, a person skilled in the art can determine the appropriate parameters for aligning sequences. When screening protein mutants, obtaining an amino acid sequence that has at least a certain percentage (e.g., 75%, 80%, 85%, 90%, 95% or 99%) identity with the wild-type protein sequence is a common method in the art.

The term "construct" refers to a genetic vector that contains a specific nucleic acid sequence and is capable of transferring the target nucleic acid sequence into a host cell to obtain a recombinant cell. According to an example of the present invention, the form of the construct is not particularly limited, and includes but is not limited to at least one of a plasmid, a phage, an artificial chromosome, a cosmid and a virus, preferably a plasmid.

The term "protein pore" is a transmembrane protein structure that defines a channel or pore that allows molecules and ions to translocate from one side of a membrane to the other side. "Nanopore" is a protein pore in which the minimum diameter of the channel through which molecules or ions pass is at nanometer level (10⁻⁹ meter). The protein pore can be a transmembrane protein pore. The transmembrane protein structure of the protein pore can be monomeric or oligomeric in nature. Typically, the pore comprises a plurality of polypeptide subunits arranged around a central axis to form a protein-lined channel extending substantially perpendicular to the membrane in which the nanopore resides.

The term "rate-controlling protein" refers to a protein that can control the speed at which a target analyte (e.g., a polynucleotide) moves relative to a protein pore (e.g., slows down its movement speed) so that this speed enables the detection of the presence, absence, or one or more characteristics of the target analyte (e.g., sequencing polynucleotide). The protein pore is used in conjunction with the rate-controlling protein to characterize the target analyte. In one example, the rate-controlling protein slows down the speed at which the polynucleotide passes through the protein pore to achieve sequencing.

The term "amphiphilic molecule" refers to a molecule having at least one hydrophilic portion and at least one lipophilic portion, for example, a phospholipid has both hydrophilicity and lipophilicity.

As described above, the present invention aims to provide a novel porin BCP34 and mutant thereof. Porin BCP34 is a nonameric protein, and its monomer sequence is mined from a metagenomic database of the Mariana Trench at a depth of 11,000 meters. The amino acid sequence of the wild-type porin BCP34 monomer is SEQ ID NO: 1, and the DNA sequence is SEQ ID NO: 2. The sequence as set forth in SEQ ID NO: 1 is MKRFIAFAVSMTLVGCASFSPPKGQTSIRDRAQPLSATVTRNALTKLPPPLAPIPAAVYNI KDQTGQYKPSPSNGFSTAVMQGATSVLVKALLDSRWFIPLEREGLQNLLTERKIIRARDS KKDLSNLAAASVIIEGSIIAYDSNVRTGGAGAKYLGIGLSEQYREDQVTVNLRAINVNNG RILQSVTSTKMIFSRQLDSGAFGYIRFKKLLEIESGYSYNEPAQLCVIDAIESALIQLIYEGV VGGTWKLKNPADIDSPIFTHYAGQNGPEGQAIM, and the sequence as set forth in SEQ ID NO: 2 is ATGAAGCGCTTTATTGCCTTTGCAGTTAGCATGACCCTGGTTGGTTGTGCAA GCTTTAGCCCGCCGAAAGGTCAGACCAGCATTCGTGATCGTGCACAGCCTCTGAGTG CAACCGTTACCCGTAATGCACTGACCAAACTGCCTCCTCCTCTGGCACCTATTCCGG CAGCAGTTTACAATATCAAGGATCAGACCGGTCAGTATAAACCGAGCCCTAGTAAT GGTTTTAGCACCGCAGTTATGCAGGGTGCAACCAGCGTTCTGGTTAAAGCATTACTG GATAGCCGTTGGTTTATTCCGCTGGAACGTGAAGGTCTGCAGAATCTGCTGACCGAA CGCAAAATTATTCGTGCACGCGACAGCAAAAAAGATCTGAGCAATCTGGCAGCAGC AAGCGTTATTATTGAGGGTAGCATCATCGCCTATGATAGCAATGTTCGTACCGGTGG TGCAGGTGCAAAATATCTGGGTATTGGTCTGAGCGAACAGTATCGTGAAGATCAGG TTACCGTTAATCTGCGCGCAATCAATGTTAACAACGGCCGTATTCTGCAGAGCGTTA CCAGCACCAAAATGATTTTTAGCCGTCAGCTGGATAGCGGTGCATTTGGTTATATTC GCTTCAAGAAGCTGCTGGAGATTGAAAGCGGTTACAGCTATAATGAACCGGCACAG CTGTGTGTTATTGATGCAATTGAGAGCGCACTGATTCAGCTGATTTATGAGGGTGTT GTTGGTGGTACCTGGAAACTGAAAAATCCGGCCGATATTGATAGCCCGATCTTTACC CATTATGCCGGTCAGAATGGTCCTGAAGGTCAGGCAATTATGTAA.

Therefore, in the first aspect, the present invention provides a porin monomer, wherein the porin monomer is a protein having an amino acid sequence as set forth in SEQ ID NO: 1 or a mutant thereof having the function of porin monomer.

In one embodiment, the mutant has an amino acid sequence having an identity of at least 70%, 75%, 80%, 85%, 90%, 95% or at least 99% to the amino acid sequence of SEQ ID NO:1 and having the function of a porin monomer, or an amino acid sequence having one or more mutations in the sensor region (S71, N74, G75, F76), transmembrane region (E162, R196, S200, S216), inlet, barrel wall and outlet (K62, D63, R103, E104, E112, R113, K114, R117, R119, D120, K122, D124, K154, R165, D199, R207, K209, K210, E213, E215) of porin BCP34 monomer; preferably, the mutations comprise:
in the sensor region:
the mutations at the S71 position comprise substitutions to G, A or T;
the mutations at the N74 position comprise substitutions to G, A, S or T;
the mutations at the G75 position comprise substitutions to A, S, T, N or Q;
the mutations at the F76 position comprise substitutions to G, A, S, T, N or Q;
in the transmembrane region:
   the mutations at the E162 position comprise substitutions to A, G, V, L, I, Y, F or W;
   the mutations at the R196 position comprise substitutions to A, G, V, L, I, Y, F or W;
   the mutations at the S200 position comprise substitutions to A, G, V, L, I, Y, F or W;
   the mutations at the S216 position comprise substitutions to A, G, V, L, I, Y, F or W.
   at the inlet:
      the mutations at the K62 position comprise substitutions to A, G, S, T, N or Q;
      the mutations at the D63 position comprise substitutions to K, R, A, G, S, T, N or Q;
      the mutations at the R103 position comprise substitutions to A, G, S, T, N or Q;
      the mutations at the E104 position comprise substitutions to K, R, A, G, S, T, N or Q;
      the mutations at the E112 position comprise substitutions to K, R, A, G, S, T, N or Q;
      the mutations at the R113 position comprise substitutions to A, G, S, T, N or Q;
      the mutations at the K114 position comprise substitutions to A, G, S, T, N or Q;
      the mutations at the R117 position comprise substitutions to A, G, S, T, N or Q;
      the mutations at the R119 position comprise substitutions to A, G, S, T, N or Q;
      the mutations at the D120 position comprise substitutions to K, R, A, G, S, T, N or Q;
      the mutations at the K122 position comprise substitutions to A, G, S, T, N or Q;
      the mutations at the D124 position comprise substitutions to K, R, A, G, S, T, N or Q;
      at the inner wall of barrel:
         the mutations at the K154 position comprise substitutions to A, G, S, T, N or Q;
         the mutations at the R165 position comprise substitutions to A, G, S, T, N or Q;
         the mutations at the D199 position comprise substitutions to A, G, S, T, N or Q;
         the mutations at the E213 position comprise substitutions to A, G, S, T, N or Q;
         the mutation at the E215 position comprise substitutions to A, G, S, T, N or Q.
         at the outlet:
            the mutations at the R207 position comprise substitutions to A, G, S, T, N, Q, D or E;
            the mutations at the K209 position comprise substitutions to A, G, S, T, N, Q, D or E;
            the mutations at the K210 position comprise substitutions to A, G, S, T, N, Q, D or E.

In the present invention, wild-type BCP34 refers to a nonameric protein composed of protein monomers having an amino acid sequence as set forth in SEQ ID NO: 1, and BCP34 mutant refers to a protein containing a mutant protein monomer. A BCP34 mutant can be a heptamer, octamer, nonamer, decamer, undecamer, and the number of mutant protein monomers in the mutant is not limited. For example, a heptamer BCP34 mutant can comprise 3 wild-type protein monomers and 4 mutant protein monomers; or, a decamer BCP34 mutant may comprise 5 wild-type protein monomers and 5 mutant protein monomers.

In a second aspect, the present invention provides a construct, wherein the construct comprises 7 to 11 porin monomers as described in the first aspect; preferably, the construct comprises 9 porin monomers as described in the first aspect.

In a third aspect, the present invention provides a protein pore, wherein the protein pore comprises 7 to 11 porin monomers as described in the first aspect; preferably, the protein pore comprises 9 porin monomers as described in the first aspect.

In one embodiment, the porin monomers in the construct and the porin monomers in the protein pore are covalently linked or non-covalently polymerized.

In a fourth aspect, the present invention provides a membrane, wherein the membrane has the protein pore as described in the third aspect.

In one embodiment, the membrane is an amphiphilic layer, i.e., a monolayer or bilayer formed by amphiphilic molecule.

In a preferred embodiment, the amphiphilic molecule is a phospholipid.

In a fifth aspect, the present invention provides a complex for characterizing a target analyte, wherein the complex comprises the protein pore as described in the third aspect and a rate-controlling protein used in conjunction with the protein pore.

In the sixth aspect, the present invention provides a nucleic acid, wherein the nucleic acid encodes the pore protein monomer as described in the first aspect, the construct as described in the second aspect, the protein pore as described in the third aspect, or the complex as described in the fifth aspect.

In the seventh aspect, the present invention provides a vector or host cell, wherein the vector or host cell comprises the nucleic acid as described in the sixth aspect.

In the eighth aspect, the present invention provides a method for producing protein pore or polypeptide thereof, comprising transforming a host cell with the vector as described in the seventh aspect, and inducing the host cell to express the protein pore or polypeptide thereof.

In the ninth aspect, the present invention provides a method for determining the presence, absence or one or more characteristics of a target analyte, the method comprising:
a. allowing the target analyte to contact a protein pore on the membrane as described in the fourth aspect, or the complex as described in the fifth aspect or a protein pore in the complex, so that the target analyte moves relative to the protein pore;
b. obtaining one or more measured values when the target analyte moves relative to the protein pore, thereby determining the presence, absence, or one or more characteristics of the target analyte.

In one embodiment, the target analyte interacts with the protein pore so that the target analyte moves relative to the protein pore.

In a tenth aspect, the present invention provides a kit for determining the presence, absence or one or more characteristics of a target analyte, wherein the kit comprises the construct as described in the second aspect, the protein pore as described in the third aspect, the membrane as described in the fourth aspect, the complex as described in the fifth aspect, the nucleic acid as described in the sixth aspect, or the vector or host cell as described in the seventh aspect.

In the eleventh aspect, the present invention provides a device for determining the presence, absence or one or more characteristics of a target analyte, wherein the device comprises the protein pore as described in the third aspect, the membrane as described in the fourth aspect, or the complex as described in the fifth aspect.

The above target analyte includes, but is not limited to, polysaccharide, amino acid, peptide, nucleotide, oligonucleotide, polynucleotide, dye, drug, diagnostic agent, explosive, and environmental pollutant.

### Examples

It should be noted that the terms used in the description of the present invention are only for the purpose of describing specific examples and are not intended to limit the present invention. The contents of the present invention as described above and the following detailed description are only for the purpose of specifically explaining the present invention and are not intended to limit the present invention in any way. Without departing from the spirit and purpose of the present invention, the scope of the present invention is determined by the appended claims.

### Prediction of structure of wild-type BCP34 using Alphafold2 multimer

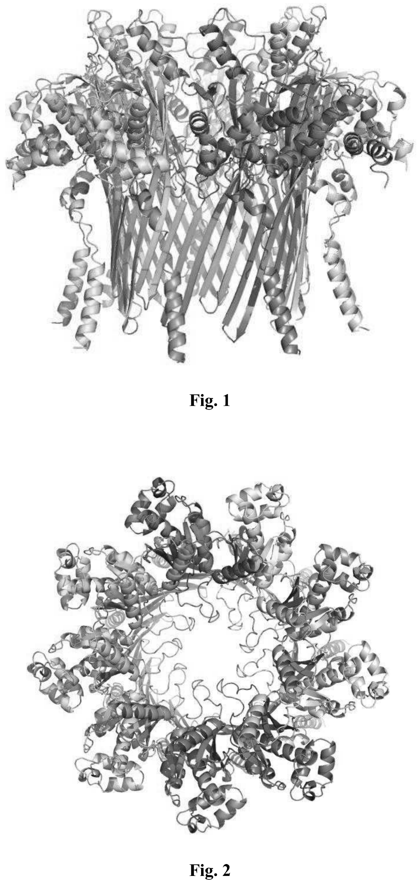

The amino acid sequence of the wild-type porin BCP34 monomer was derived from the Mariana Trench Metagenome Database. The nonamer structure of wild-type BCP34 was predicted using Alphafold2 multimer. The structure prediction results were shown in Fig. 1 and Fig. 2, where Fig. 1 and Fig. 2 are the side view and top view of the BCP34 nonamer structure, respectively.

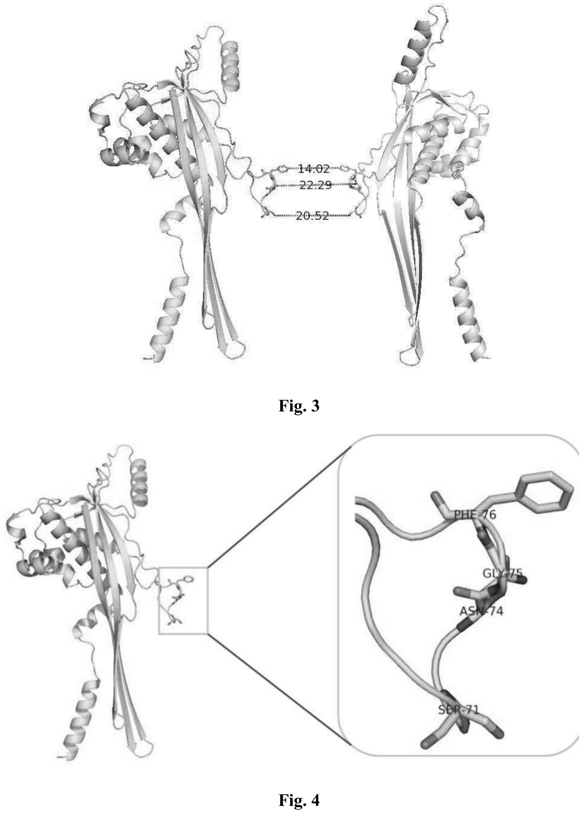

The amino acid composition of the sensor region could play an important role in the generation of current signals, so we tried to target mutations in the sensor region of BCP34 mutant. Fig. 3 and Fig. 4 showed the side chain structures of the important amino acids in the sensor region of the predicted structure of BCP34, showing that the four amino acids in the amino acid side chains were S71, N74, G75, and F76, respectively.

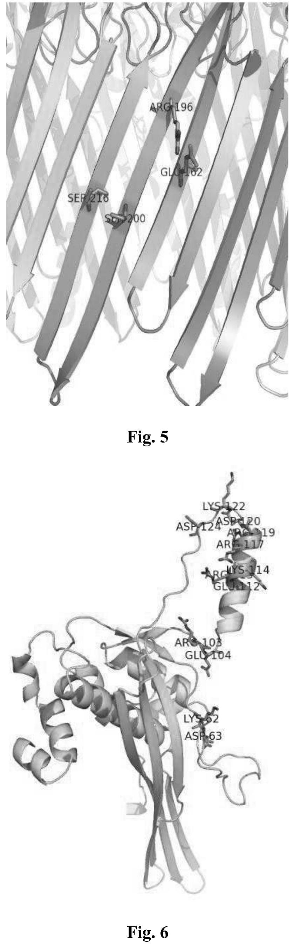

In the transmembrane region of pore protein, the amino acids facing toward the membrane were preferred hydrophobic amino acids, while charged amino acids and polar amino acids could affect the efficiency of pore insertion. Fig. 5 showed the four charged and polar amino acids facing the membrane in the transmembrane region of BCP34, namely E162, R196, S200, S216.

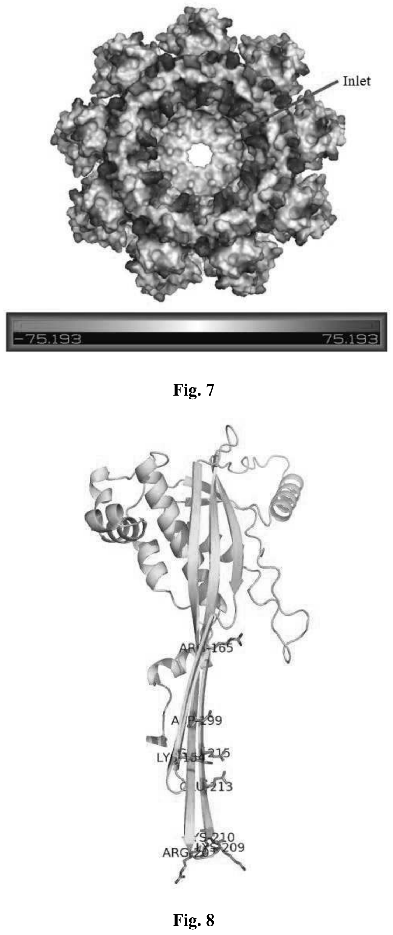

Since nucleic acids are negatively charged, if the amino acids at the inlet of the pore protein are positively charged, the library capture rate of nanopore sequencing could be increased. Otherwise, the library capture rate could be reduced. At the same time, the strength of the binding between the library and the pore protein had a certain influence on the sequencing speed and sequencing current signal. Fig. 6 showed some important amino acids in the inlet region of the BCP34 monomer, which were K62, D63, R103, E104, E112, R113, K114, R117, R119, D120, K122, and D124. Fig. 7 showed the charge distribution of the inlet region of the predicted structure of BCP34, and it could be seen that there was a large amount of positive charge in the inlet region of the BCP34 pore protein, and this feature supported its stronger library capture ability compared with the existing technology.

At the same time, some amino acids on the inner wall and outlet of barrel could have a certain influence on the analyte to pass through and leave the nanopore. Fig. 8 showed some amino acids on the inner wall and outlet of barrel of the BCP34 monomer that could affect the smooth perforation of the analyte to pass through the pore, especially the charged amino acids, which were K154, R165, D199, R207, K209, K210, E213 and E215, respectively.

### Construction of expression vectors for wild-type BCP34 and mutant 1 thereof

The amino acid composition of the sensor region played a decisive role in the generation of current signals in sequencing. Therefore, we constructed mutant 1 targeting the sensor region of BCP34 monomer, with mutation sites of N74S, G75N and F76A, the amino acid sequence was SEQ ID NO:3, the DNA sequence was SEQ ID NO:4. The sequence as set forth in SEQ ID NO:3 was MKRFIAFAVSMTLVGCASFSPPKGQTSIRDRAQPLSATVTRNALTKLPPPLA PIPAAVYNIKDQTGQYKPSPSSNASTAVMQGATSVLVKALLDSRWFIPLEREGLQNLLTE RKIIRARDSKKDLSNLAAASVIIEGSIIAYDSNVRTGGAGAKYLGIGLSEQYREDQVTVNL RAINVNNGRILQSVTSTKMIFSRQLDSGAFGYIRFKKLLEIESGYSYNEPAQLCVIDAIESA LIQLIYEGVVGGTWKLKNPADIDSPIFTHYAGQNGPEGQAIM, and the sequence as set forth in SEQ ID NO: 4 was ATGAAGCGCTTTATTGCCTTTGCAGTTAGCATGACCCT GGTTGGTTGTGCAAGCTTTAGCCCGCCGAAAGGTCAGACCAGCATTCGTGATCGTGC ACAGCCTCTGAGTGCAACCGTTACCCGTAATGCACTGACCAAACTGCCTCCTCCTCT GGCACCTATTCCGGCAGCAGTTTACAATATCAAGGATCAGACCGGTCAGTATAAAC CGGCACCGGCAAGCAATCAGAGCACCGCAGTTATGCAGGGTGCAACCAGCGTTCTG GTTAAAGCATTACTGGATAGCCGTTGGTTTATTCCGCTGGAACGTGAAGGTCTGCAG AATCTGCTGACCGAACGCAAAATTATTCGTGCACGCGACAGCAAAAAAGATCTGAG CAATCTGGCAGCAGCAAGCGTTATTATTGAGGGTAGCATCATCGCCTATGATAGCAA TGTTCGTACCGGTGGTGCAGGTGCAAAATATCTGGGTATTGGTCTGAGCGAACAGTA TCGTGAAGATCAGGTTACCGTTAATCTGCGCGCAATCAATGTTAACAACGGCCGTAT TCTGCAGAGCGTTACCAGCACCAAAATGATTTTTAGCCGTCAGCTGGATAGCGGTGC ATTTGGTTATATTCGCTTCAAGAAGCTGCTGGAGATTGAAAGCGGTTACAGCTATAA TGAACCGGCACAGCTGTGTGTTATTGATGCAATTGAGAGCGCACTGATTCAGCTGAT TTATGAGGGTGTTGTTGGTGGTACCTGGAAACTGAAAAATCCGGCCGATATTGATAG CCCGATCTTTACCCATTATGCCGGTCAGAATGGTCCTGAAGGTCAGGCAATTATGTA A. Herein, the nanoporin BCP34 mutant 1 referred to a nonamer composed of a protein monomers with an amino acid sequence of SEQ ID NO:3.

The DNA sequence of the porin BCP34 was inserted into the multicloning region of the vector pET24a by the In-fusion method after digestion with NdeI and XhoI. StrepII amino acid as a purification tag was added to the C-terminal of the amino acid sequence of wild-type BCP34 (SEQ ID NO:1), wherein the screening tag was kanamycin, and the constructed vector was named pET24a-BCP34. By using the site-directed mutagenesis method, the Agilent site-directed mutagenesis kit was used to construct the corresponding mutant 1 with the expression vector of porin BCP34 as a template.

### Cultivation and induction of wild-type BCP34 and mutant 1 strain thereof

The constructed wild-type BCP34 or its mutant 1 expression plasmid was transformed into *E*. *coli* expression strain *E. coli* BL21 (*DE3*)*,* and the bacterial solution was evenly spread on a plate containing 50 µg/mL kanamycin and cultured at 37°C overnight. On the next day, a single colony was picked and placed in 5 mL of LB medium containing 50 µg/mL kanamycin, and cultured at 37°C, 200 rpm, overnight. The above-obtained bacterial solution was inoculated into 50 mL of LB containing 50 µg/mL kanamycin at a ratio of 1:100, and cultured at 37°C, 200 rpm for 4 h. The expanded cultured bacterial solution was inoculated into 2 L of LB containing 50 µg/mL kanamycin at a ratio of 1:100, and cultured at 37°C, 200 rpm. When the OD600 value reached about 0.6 to 0.8, IPTG with a final concentration of 0.5 mM was added, and cultured at 16°C, 200 rpm, for about 16-18 h. The bacterial solution was collected by centrifugation at 8000 rpm, and the bacteria were frozen at -20°C for later use.

### Extraction and purification of recombinant wild-type BCP34 and its mutant 1 protein

### Preparation of purification buffers:

Buffer A: 20 mM Tris-HCl, 250 mM NaCl, 1% DDM, pH 8.0.
Buffer B: 20 mM Tris-HCl, 250 mM NaCl, 0.05% DDM, pH 8.0.
Buffer C: 20 mM Tris-HCl, 250 mM NaCl, 0.05% DDM, 5 mM desthiobiotin, pH 8.0.

### Purification steps:

The cells were fully resuspended at a ratio of 1 g of cells to 10 mL of Buffer A, and the cells were ultrasonically disrupted until the cell solution was clear, and rotated overnight at 4°C on a rotator. On the next day, the cells were centrifuged at 18,000 rpm for 1 h at 4°C, the supernatant was taken, filtered with a 0.22 µm filter, and stored at 4°C for later use.

After equilibrating the Strep-Tactin beads (IBA Lifesciences) column with Buffer A for 5CV using an AKTA pure chromatograph, the sample was loaded at 2 mL/min. After loading, rinsing was performed with Buffer B for 20CV, elution was performed with Buffer C, and the target protein was collected.

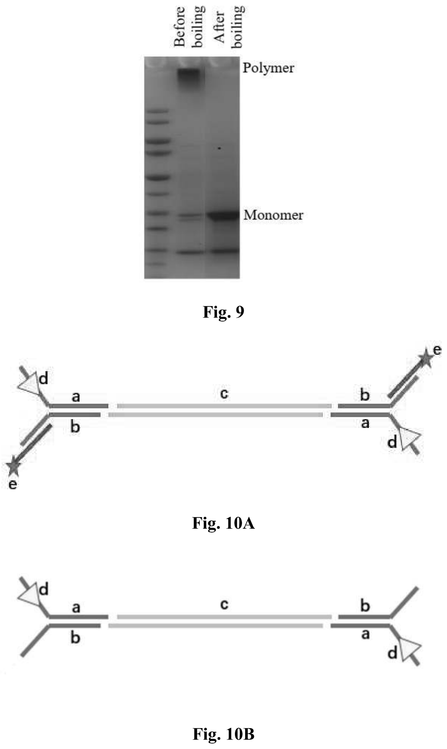

The resulting protein was concentrated to 1 mL, passed through a Superdex 6 increase 10/300GL (Cytiva) column equilibrated with Buffer B, and the protein was collected and then stored at -80°C for subsequent nanopore sequencing experiments. Fig. 9 was an SDS-PAGE image of BCP34 after purification. Lanes 2 and 3 were the gel stripes before and after boiling at 95°C, respectively. The results showed that the target protein was in a polymer state under conditions of not boiling, and in a monomer state after boiling.

### Library construction

Two partially complementary DNA strands (top strand: SEQ ID NO:5, bottom strand: SEQ ID NO:6) were annealed to form an adaptor, and then ligated to the double-stranded target fragment to be tested (SEQ ID NO:7) at room temperature using T4 DNA ligase and purified to prepare a sequencing library. The sequence of the top strand was TTTTTTTTTTTTTTTTTTTTTTTTTTTTTTTTTTTTTTTTTTTTTTTTYYYYGGTTGTTTCT GTTGGTGCTGATATTGCT (SEQ ID NO:5), where Y represented iSP18 (without base group), and the sequence as set forth in SEQ ID NO: 6 was GCAATATCAGCACCAACAGAAACAACCTTTGAGGCGAGCGGTCAA, the sequence as set forth in SEQ ID NO: 7 was TCGCGCGTTTCGGTGATGACGGTGAAAACCTCTGACACATGCAGCTCCCGGAGACG GTCACAGCTTGTCTGTAAGCGGATGCCGGGAGCAGACAAGCCCGTCAGGGCGCGTC AGCGGGTGTTGGCGGGTGTCGGGGCTGGCTTAACTATGCGGCATCAGAGCAGATTG TACTGAGAGTGCACCATATGCGGTGTGAAATACCGCACAGATGCGTAAGGAGAAAA TACCGCATCAGGCGCCATTCGCCATTCAGGCTGCGCAACTGTTGGGAAGGGCGATC GGTGCGGGCCTCTTCGCTATTACGCCAGCTGGCGAAAGGGGGATGTGCTGCAAGGC GATTAAGTTGGGTAACGCCAGGGTTTTCCCAGTCACGACGTTGTAAAACGACGGCC AGTGAATTCGAGCTCGGTACCTCGCGAATGCATCTAGATATCGGATCCCGGGCCCGT CGACTGCAGAGGCCTGCATGCAAGCTTGGCGTAATCATGGTCATAGCTGTTTCCTGT GTGAAATTGTTATCCGCTCACAATTCCACACAACATACGAGCCGGAAGCATAAAGT GTAAAGCCTGGGGTGCCTAATGAGTGAGCTAACTCACATTAATTGCGTTGCGCTCAC TGCCCGCTTTCCAGTCGGGAAACCTGTCGTGCCAGCTGCATTAATGAATCGGCCAAC GCGCGGGGAGAGGCGGTTTGCGTATTGGGCGCTCTTCCGCTTCCTCGCTCACTGACT CGCTGCGCTCGGTCGTTCGGCTGCGGCGAGCGGTATCAGCTCACTCAAAGGCGGTA ATACGGTTATCCACAGAATCAGGGGATAACGCAGGAAAGAACATGTGAGCAAAAG GCCAGCAAAAGGCCAGGAACCGTAAAAAGGCCGCGTTGCTGGCGTTTTTCCATAGG CTCCGCCCCCCTGACGAGCATCACAAAAATCGACGCTCAAGTCAGAGGTGGCGAAA CCCGACAGGACTATAAAGATACCAGGCGTTTCCCCCTGGAAGCTCCCTCGTGCGCTC TCCTGTTCCGACCCTGCCGCTTACCGGATACCTGTCCGCCTTTCTCCCTTCGGGAAGC GTGGCGCTTTCTCATAGCTCACGCTGTAGGTATCTCAGTTCGGTGTAGGTCGTTCGCT CCAAGCTGGGCTGTGTGCACGAACCCCCCGTTCAGCCCGACCGCTGCGCCTTATCCG GTAACTATCGTCTTGAGTCCAACCCGGTAAGACACGACTTATCGCCACTGGCAGCAG CCACTGGTAACAGGATTAGCAGAGCGAGGTATGTAGGCGGTGCTACAGAGTTCTTG AAGTGGTGGCCTAACTACGGCTACACTAGAAGAACAGTATTTGGTATCTGCGCTCTG CTGAAGCCAGTTACCTTCGGAAAAAGAGTTGGTAGCTCTTGATCCGGCAAACAAAC CACCGCTGGTAGCGGTGGTTTTTTTGTTTGCAAGCAGCAGATTACGCGCAGAAAAAA AGGATCTCAAGAAGATCCTTTGATCTTTTCTACGGGGTCTGACGCTCAGTGGAACGA AAACTCACGTTAAGGGATTTTGGTCATGAGATTATCAAAAAGGATCTTCACCTAGAT CCTTTTAAATTAAAAATGAAGTTTTAAATCAATCTAAAGTATATATGAGTAAACTTG GTCTGACAGTTACCAATGCTTAATCAGTGAGGCACCTATCTCAGCGATCTGTCTATT TCGTTCATCCATAGTTGCCTGACTCCCCGTCGTGTAGATAACTACGATACGGGAGGG CTTACCATCTGGCCCCAGTGCTGCAATGATACCGCGAGACCCACGCTCACCGGCTCC AGATTTATCAGCAATAAACCAGCCAGCCGGAAGGGCCGAGCGCAGAAGTGGTCCTG CAACTTTATCCGCCTCCATCCAGTCTATTAATTGTTGCCGGGAAGCTAGAGTAAGTA GTTCGCCAGTTAATAGTTTGCGCAACGTTGTTGCCATTGCTACAGGCATCGTGGTGT CACGCTCGTCGTTTGGTATGGCTTCATTCAGCTCCGGTTCCCAACGATCAAGGCGAG TTACATGATCCCCCATGTTGTGCAAAAAAGCGGTTAGCTCCTTCGGTCCTCCGATCG TTGTCAGAAGTAAGTTGGCCGCAGTGTTATCACTCATGGTTATGGCAGCACTGCATA ATTCTCTTACTGTCATGCCATCCGTAAGATGCTTTTCTGTGACTGGTGAGTACTCAAC CAAGTCATTCTGAGAATAGTGTATGCGGCGACCGAGTTGCTCTTGCCCGGCGTCAAT ACGGGATAATACCGCGCCACATAGCAGAACTTTAAAAGTGCTCATCATTGGAAAAC GTTCTTCGGGGCGAAAACTCTCAAGGATCTTACCGCTGTTGAGATCCAGTTCGATGT AACCCACTCGTGCACCCAACTGATCTTCAGCATCTTTTACTTTCACCAGCGTTTCTGG GTGAGCAAAAACAGGAAGGCAAAATGCCGCAAAAAAGGGAATAAGGGCGACACGG AAATGTTGAATACTCATACTCTTCCTTTTTCAATATTATTGAAGCATTTATCAGGGTT ATTGTCTCATGAGCGGATACATATTTGAATGTATTTAGAAAAATAAACAAATAGGG GTTCCGCGCACATTTCCCCGAAAAGTGCCACCTGACGTCTAAGAAACCATTATTATC ATGACATTAACCTATAAAAATAGGCGTATCACGAGGCCCTTTCGTC. The sequencing library was incubated with helicase BCH105 (SEQ ID NO: 8) at 25°C for 1 h (molar concentration ratio 1:8). After cross-linking and purification, a sequencing library containing BCH105 motor protein was formed (as shown in Fig. 10A). The sequence as set forth in SEQ ID NO: 8 was MKHDLSSATEEQRYIFTDFIKQAQLALKGDSDYEVFAIQGFAGSGKTWLSALIIDELLEL GMKVAVTSPTHKAVRVSLNMLKNNGIDTNSPLMYPGTIHHFLNLKLDHGFADDGTACN VTTKAKLVVNKFNEALEYVDVLIVDEASMVSGELYDHALKTLGDRAKIILFIGDSYQLL PVDDEDSSIFLKDDIFHYKLTKVVRQAEDNIIIAKSQELIKAMDQKTYYPSVNDYFVNITE DTEGIKLLKSNVELFELYFSDFKDKMTGAYTNKVVNQFNEYIRYTLYQETKFIADKDEL VFQETYTDSKGNIIVSNGEIIEVATAKLTTDIDKFKIWKIVSKKNELGEAVRFNVLDPSSY NEFNDLLDKYCADAKIAKGYDRSKAWKKYFKLKEKYAKVRYNFSSTIHKLQGSTYQN MYFDMRGLDYFYRMNRDNVLRLVYVGITRASDQVFILQD. During sequencing, the sequencing library can further complementarily pair and bind to single-stranded DNA with cholesterol (SEQ ID NO:9, cholesterol was connected to the 5' end of DNA) to form a structure as shown in Fig. 10B. The sequence shown in SEQ ID NO:9 was TTGACCGCTCGCCTC.

### Construction of nanopore biosensor using wild-type BCP34 and its mutant 1

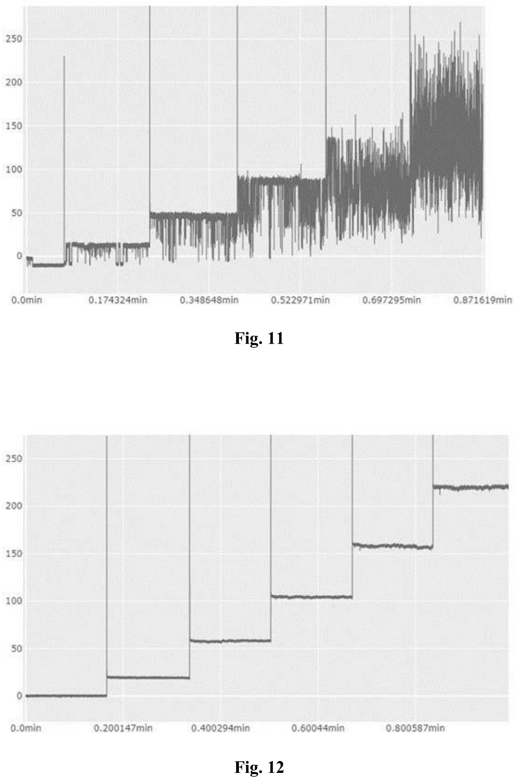

Single-channel nanopore current measurement was based on an amplifier of digital device. Ag/AgCl electrodes were immersed in sequencing buffer (components included: 0.47 M KCl, 25 mM HEPES, 1 mM EDTA, 5 mM ATP, 25 mM MgCl2, pH7.6), and the electrodes were located in the cis and trans regions of the electrolytic cell, respectively. Sequencing library and nanopore reagents were added to the cis region. After the nanopore protein was diluted to a certain multiple using 1xPBS buffer, a single nanopore was inserted into a phospholipid bilayer composed of 1,2-diphytanoyl-sn-glycero-3-phosphocholine (DPhPC) under the action of an external electric field to form a nanopore biosensor. An external voltage was applied to obtain the current amplitude value of single pore protein. Fig. 11 and Fig. 12 were the nanopore biosensor currents of wild-type BCP34 and mutant 1 when voltages of 0.02V, 0.04V, 0.10V, 0.14V and 0.18V were applied, respectively. The results showed that the open-pore current noise of wild-type BCP34 was large, while the open-pore current noise of mutant 1 was small, indicating that the mutation in the sensor region could effectively suppress the open-pore current noise.

### DNA sequencing using wild-type BCP34 and its mutant 1

The sequencing library containing pUC57 sequence and single-stranded DNA with cholesterol (SEQ ID NO:9) were mixed with the sequencing buffer and added to the nanopore biosensor. After applying an external voltage of 0.14V or 0.18V, it was observed that the DNA was captured by the nanopore, generating a characteristic blockade current amplitude value. As the DNA moved through the nanopore, the current amplitude value changed. Different DNA sequences produced different blockade current amplitude values. Single-stranded DNA with cholesterol could bind to the phospholipid bilayer, which helped the nanopore capture the sequencing library and reduce the amount of sequencing library loading.

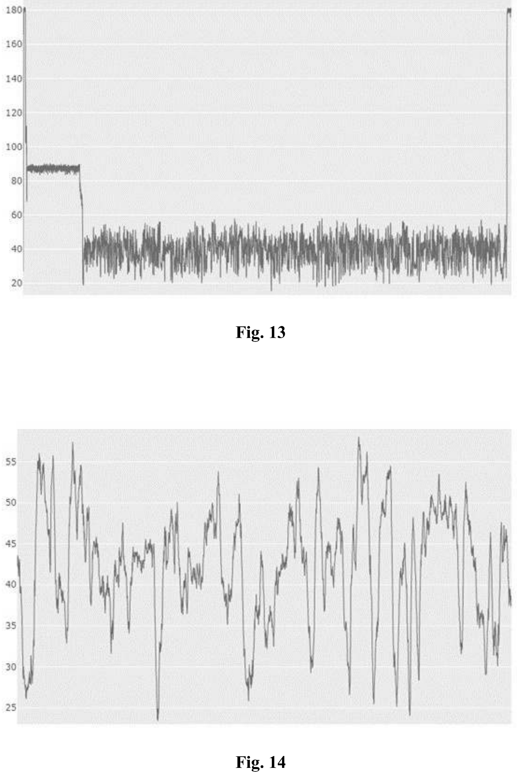

Under the action of an external voltage of 0.14V applied, the current trace change of the library DNA passing through the wild-type BCP34 could not be identified due to excessive noise. The current trace change of the library DNA passing through mutant 1 was shown in Fig. 13. The results showed that mutant 1 could effectively reduce sequencing current noise. Fig. 14 showed the local details of the current trace when mutant 1 performed DNA sequencing. The results showed that the mutation in the porin BCP34 sensor region could improve the properties of the nanopore sequencing current and had the potential to improve sequencing accuracy.

### BCP34 mutant 1 showing stronger library capture ability than the prior art

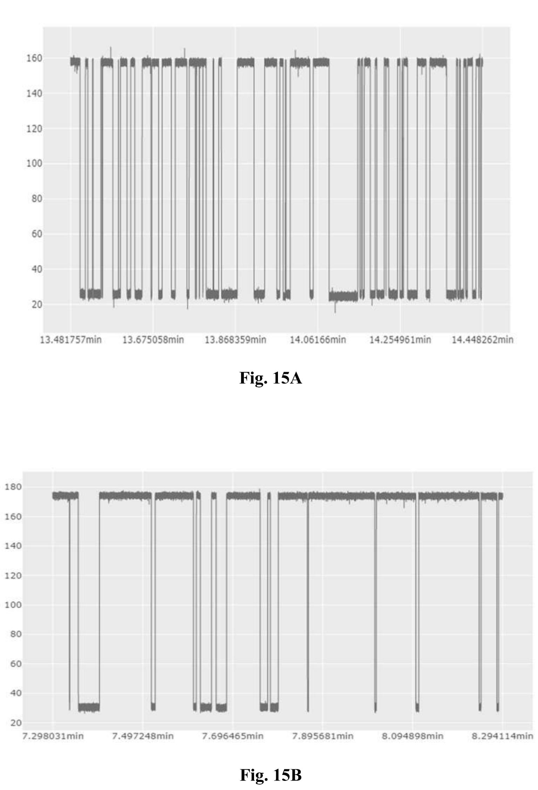

The nanopore protein BCP34 mutant 1 and the prior art porin CSGG-Y51A/F56Q were respectively embedded in nanopores. After the embedding was completed, a single pore was selected based on the opening current. 3µl of 10µM QCDNA (SEQ ID NO: 10) was added to the cis region of electrolytic cell. The sequence as shown in SEQ ID NO: 10 was TTTTTTTTTTGGTGGTGTGGTTGG. QCDNA could form a G4 structure, which can be pulled apart under the action of an electric field force. After applying an external voltage of 0.14V, it was observed that QCDNA was captured by the nanopore, generating a blocking current; after the QCDNA completely passed through the nanopore, the current returned to the opening current, and then new QCDNA was captured. Fig. 15A showed the capture effect of nanoporin BCP34 mutant 1 on QCDNA at 0.14V; and Fig. 15B showed the capture effect of nanoporin CSGG-Y51A/F56Q of the prior art on QCDNA at 0.14V. It could be seen that the capture ability of BCP34 mutant 1 on QCDNA was significantly stronger than that of the prior art porin CSGG-Y51A/F56Q.

In view of the strong capture ability of porin BCP34 mutant 1 for the library, when it was used for nanopore sequencing, it would have the potential to increase sequencing throughput or reduce library loading as compared with the prior art.

Finally, it should be noted that the above examples are only used to illustrate the technical solutions of the present invention, rather than to limit them; although the present invention is described in detail with reference to the aforementioned examples, ordinary technicians in the field should understand that they can still modify the technical solutions recorded in the aforementioned examples, or replace some of the technical features therein by equivalent ones; and these modifications or replacements do not make the essence of the corresponding technical solutions deviate from the spirit and scope of the technical solutions of the examples of the present invention.

## Claims

1. A porin monomer, **characterized in that** the porin monomer is a protein having an amino acid sequence as set forth in SEQ ID NO:1 or a mutant thereof having the function of porin monomer.

2. The porin monomer according to claim 1, **characterized in that** the mutant has an amino acid sequence having an identity of at least 70%, 75%, 80%, 85%, 90%, 95% or at least 99% as compared to the amino acid sequence as set forth in SEQ ID NO: 1 and having the function of porin monomer.

3. The porin monomer according to claim 1, **characterized in that** the mutant comprises a mutation of amino acid at one or more positions corresponding to S71, N74, G75 and F76 of SEQ ID NO:1; preferably,
the mutations at the S71 position comprise substitutions to G, A or T;
the mutations at the N74 position comprise substitutions to G, A, S or T;
the mutations at the G75 position comprise substitutions to A, S, T, N or Q;
the mutations at the F76 position comprise substitutions to G, A, S, T, N or Q.

4. The porin monomer according to claim 1, **characterized in that** the mutant comprises a mutation of amino acid at one or more positions corresponding to E162, R196, S200 and S216 of SEQ ID NO: 1; preferably,
the mutations at the E162 position comprise substitutions to A, G, V, L, I, Y, F or W;
the mutations at the R196 position comprise substitutions to A, G, V, L, I, Y, F or W;
the mutations at the S200 position comprise substitutions to A, G, V, L, I, Y, F or W;
the mutations at the S216 position comprise substitutions to A, G, V, L, I, Y, F or W.

5. The porin monomer according to claim 1, **characterized in that** the mutant comprises a mutation of amino acid at one or more positions corresponding to K62, D63, R103, E104, E112, R113, K114, R117, R119, D120, K122, D124, K154, R165, D199, R207, K209, K210, E213 and E215 of SEQ ID NO: 1; preferably,
the mutations at the K62 position comprise substitutions to A, G, S, T, N or Q;
the mutations at the D63 position comprise substitutions to K, R, A, G, S, T, N or Q;
the mutations at the R103 position comprise substitutions to A, G, S, T, N or Q;
the mutations at the E104 position comprise substitutions to K, R, A, G, S, T, N or Q;
the mutations at the E112 position comprise substitutions to K, R, A, G, S, T, N or Q;
the mutations at the R113 position comprise substitutions to A, G, S, T, N or Q;
the mutations at the K114 position comprise substitutions to A, G, S, T, N or Q;
the mutations at the R117 position comprise substitutions to A, G, S, T, N or Q;
the mutations at the R119 position comprise substitutions to A, G, S, T, N or Q;
the mutations at the D120 position comprise substitutions to K, R, A, G, S, T, N or Q;
the mutations at the K122 position comprise substitutions to A, G, S, T, N or Q;
the mutations at the D124 position comprise substitutions to K, R, A, G, S, T, N or Q;
the mutations at the K154 position comprise substitutions to A, G, S, T, N or Q;
the mutations at the R165 position comprise substitutions to A, G, S, T, N or Q;
the mutations at the D199 position comprise substitutions to A, G, S, T, N or Q;
the mutations at the R207 position comprise substitutions to A, G, S, T, N, Q, D or E;
the mutations at the K209 position comprise substitutions to A, G, S, T, N, Q, D or E;
the mutations at the K210 position comprise substitutions to A, G, S, T, N, Q, D or E;
the mutations at the E213 position comprise substitutions to A, G, S, T, N or Q;
the mutation at the E215 position comprise substitutions to A, G, S, T, N or Q.

6. A construct, **characterized in that** the construct comprises 7 to 11 porin monomers according to any one of claims 1 to 5; preferably, the construct comprises 9 porin monomers according to any one of claims 1 to 5.

7. The construct according to claim 6, **characterized in that** the porin monomers are covalently linked or non-covalently polymerized.

8. A protein pore, **characterized in that** the protein pore comprises 7 to 11 porin monomers according to any one of claims 1 to 5; preferably, the protein pore comprises 9 porin monomers according to any one of claims 1 to 5.

9. The protein pore according to claim 8, **characterized in that** the porin monomers are covalently linked or non-covalently polymerized.

10. A membrane, **characterized in that** the membrane has the protein pore according to claim 8 or 9.

11. The membrane according to claim 10, **characterized in that** the membrane is an amphiphilic layer, that is, a monolayer or bilayer formed by an amphiphilic molecule.

12. The membrane according to claim 11, **characterized in that** the amphiphilic molecule is a phospholipid.

13. A complex for characterizing a target analyte, **characterized in that** the complex comprises the protein pore according to claim 8 or 9 and a rate-controlling protein used in conjunction with the protein pore.

14. A nucleic acid, **characterized in that** the nucleic acid encodes the porin monomer according to any one of claims 1 to 5, the construct according to claim 6 or 7, the protein pore according to claim 8 or 9, or the complex according to claim 13.

15. A vector or host cell, **characterized in that** the vector or host cell comprises the nucleic acid according to claim 14.

16. A method for producing a protein pore or polypeptide thereof, **characterized by**, comprising transforming a host cell with the vector according to claim 15 to induce the host cell to express the protein pore or polypeptide thereof.

17. Use of the porin monomer according to any one of claims 1 to 5, the construct according to claim 6 or 7, the protein pore according to claim 8 or 9, the membrane according to any one of claims 10 to 12, the complex according to claim 13, the nucleic acid according to claim 14, or the vector or host cell according to claim 15 in single molecule sequencing, wherein the single molecule includes but is not limited to DNA, RNA, polysaccharide and polypeptide.

18. Use of the porin monomer according to any one of claims 1 to 5, the construct according to claim 6 or 7, the protein pore according to claim 8 or 9, the membrane according to any one of claims 10 to 12, the complex according to claim 13, the nucleic acid according to claim 14, or the vector or host cell according to claim 15 in detecting the presence, absence or one or more characteristics of a target analyte or preparing a product for detecting the presence, absence or one or more characteristics of a target analyte.

19. A method for determining the presence, absence or one or more characteristics of a target analyte, **characterized in that** the method comprises:
a. contacting the target analyte with a protein pore on the membrane according to any one of claims 10 to 12, or the complex according to claim 13 or a protein pore in the complex, so that the target analyte moves relative to the protein pore;
b. obtaining one or more measurement values when the target analyte moves relative to the protein pore, thereby determining the presence, absence or one or more characteristics of the target analyte.

20. The method according to claim 19, **characterized in that** the target analyte interacts with the protein pore so that the target analyte moves relative to the protein pore.

21. A kit for determining the presence, absence or one or more characteristics of a target analyte, **characterized in that** the kit comprises the construct according to claim 6 or 7, the protein pore according to claim 8 or 9, the membrane according to any one of claims 10 to 12, the complex according to claim 13, the nucleic acid according to claim 14, or the vector or host cell according to claim 15.

22. A device for determining the presence, absence or one or more characteristics of a target analyte, **characterized in that** the device comprises the protein pore according to claim 8 or 9, the membrane according to any one of claims 10 to 12, or the complex according to claim 13.

23. The use, method, kit or device according to any one of claims 18 to 22, **characterized in that** the target analyte comprises a polysaccharide, an amino acid, a peptide, a nucleotide, an oligonucleotide, a polynucleotide, a dye, a drug, a diagnostic agent, an explosive, or an environmental pollutant.
